# EUROPEAN PATENT SPECIFICATION

(11) **EP 1 983 947 B1**
(45) Date of publication and mention of the grant of the patent: **19.05.2010**
(21) Application number: 04808807.4
(22) Date of filing: 21.12.2004
(51) Int. Cl.: A61F 5/02

(54) **BRACE FOR TREATMENT OF SPINAL DEFORMITIES**
SCHIENE ZUR BEHANDLUNG VON WIRBELSÄULENVERFORMUNGEN
APPAREIL ORTHOPEDIQUE DE TRAITEMENT DES DEFORMATIONS SPINALES

(43) Date of publication of application: 29.10.2008
(73) Proprietor: Van Loon, Petrus J.M., 6861 EE Oosterbeek (NL)
(72) Inventor: Van Loon, Petrus J.M., 6861 EE Oosterbeek (NL)
(74) Representative: Hatzmann, Martin
(86) International application number: PCT/NL2004/000891
(87) International publication number: WO 2006/068459

(56) References cited:
- US-A- 2 808 050
- US-A- 3 220 407
- US-A- 4 285 336

## Description

The invention relates to a brace for treatment of spinal deformities of a patient, such as Idiopathic Scoliosis, Spondylolisthesis, and/or Idiopathic Kyphosis.

In humans, being upright walking animals, two main forms of spinal deformities are known in which the spinal column develops curvatures beyond ranges to be considered as normal.

In a normal adult spine viewed from aside, curvatures are denominated as lordosis in the cervical spine , kyphosis in the thoracic spine, lordosis again in the lumbar spine and a kyphotic form in the sacrum.

Abnormal curvatures, becoming visible during growth are usually called hyperkyphosis if the kyphosis in the thoracal or dorsal spine exceeds 40° at the end of childhood. Looking from the rear to the spine, any deformation with a curvature to the side (one or more) is called scoliosis. In the latter being a three-dimensional deformity, an important part of it is in a rotational direction. It's that part that really creates the pathonogmonic rib hump or Gibbus Costarum.

Until now, any kyphotic deformity or scoliosis, starting during growth and being progressive is called idiopathic, without clear cause. There is no direct linkage in literature between Idiopathic Scoliosis and Idiopathic Kyphosis, mostly called Morbus Scheuermann, although presence of some scoliosis up to 20 ° in many kyphotic deformities is described. There is no mention of proven related causes in the two main deformities in childhood. Generally, it is believed that in Idiopathic Scoliosis and Kyphosis, the cause of the deformity is to be sought at the level of the apex of the curve, or completely outside the spinal column and its contents.

Both Idiopathic Scoliosis and Idiopathic Kyphosis can be progressive during growth and can give serious deformities at the end of growth.

Treatment of both conditions until now is usually dependent on the moment of discovery, severity and the interest of the physicians dealing with children having these deformities.

Treatment options vary between neglecting, controlled neglecting, postural exercises, and different forms of bracing or in severe cases corrective surgery. The following braces are known for treating spinal deformity:
- the Milwaukee brace (Moe, Blount), comprising a stiff part around the pelvis with extension poles up to the chin with the purpose to create extension of the spine and the curves;
- the Boston Brace (hall, Miller), comprising plastic modules fitting the thoraco-lumbar-sacral spine and adapted with cuttings and polsters to correct curves also in a rotational direction by three point fixation;
- the Cheneau brace, comprising a plastic moulded brace using corrected positions as base; It comprises a whole series of denominated pressure points, all with areas of releave at the opposite side of the body
- the Wilmington brace, comprising a custom-moulded plastic brace, in which pads are positioned to create rotational correction;
- the Charleston brace, comprising a plastic moulded brace with overcorrection of the curves in a lateral bending way (this brace is designed for use during the night only);
- the TriaC brace (Veldhuizen), which has a movable construct, giving three dimensional mainly rotational correction by constantly applied generated by springs; and
- the Weiss brace, which focuses on creating lordosis in the lumbar spine, thereby claiming to correct rotational curves as well. Braces, similar to the Weiss brace, are shown in US 2004/0167449 A1 and WO 00/66047.

Most of these braces have to be worn 24 hours a day. An advantage of a spinal brace is, that its utilisation may prevent surgery to correct a spinal deformity. However, a disadvantage of many of the known braces is, that they are very uncomfortable during use. The application of some braces may even lead to pain and distress, sometimes even pressure ulcers when being worn. This is, because most of the known braces are adapted to apply relatively high rotational counter pressures to fixate the deformities first, and to 'push' the deformities out of the spine thereafter. Moreover, most of the known braces are not able to provide a lasting true correction of the spinal deformations, especially in scoliosis. Therefore, in the last couple of years, many doctors have decided not to prescribe the use of a brace, but to leave the medical disorder untreated, leading to children having spines that become more and more disoriented during growth or prepare them for corrective surgery.

US 2,808,050 discloses a surgical brace employing the three-point leverage principle.

US 3,220,407 relates to a hyperextension back brace such as used in producing hyperextension of the lower thoracic and lumbar regions.

US 4,285,336 describes an orthopedic brace for treatment of idiopathic scoliosis or curvature of the spine.

The present invention aims to solve the above-mentioned problems. Particularly, the invention aims to provide a brace for treatment of spinal deformities of a patient, such as Idiopathic Scoliosis and/or Idiopathic Kyphosis or other deformities, which brace may provide a standstill of further development of the deformity, or even a certain restoration of the spine. Also, the invention aims to provide a brace that is comfortable during use.

According to the invention, there is provided a brace for treatment of spinal deformities of a patient, such as Idiopathic Scoliosis, Spondylolisthesis and/or Idiopathic Kyphosis, the brace at least comprising a posterior support and a anterior support arranged to apply pressure to a posterior area and at least one anterior counter-area respectively, the brace being characterised in that the posterior support is a thoracolumbar area support arranged to apply a lordotic pressure to a thoracolumbar area only, and to apply no or relatively little pressure to other spinal areas than the thoracolumbar area, when the brace is worn by the patient.

The brace according to the invention is adapted to apply pressure to the thoracolumbar area of the spine during use. This area lies at the thoracolumbar junction of the spine, between the lumbar spine part and thoracic spine part (by usual convention between vertebrae Th10-L2). It is this junction that forms the changeover between the lordotic lower spine part and kyphotic upper back part of a normal spine in its neutral position.

The brace according to the invention is comfortable during use. This is, because the brace applies pressure to said thoracolumbar area, and preferably mainly to that area, wherein the brace is not arranged to apply large treatment pressures onto deformed lumbar and/or thoracic spine parts for pushing these parts back towards 'usual' positions. Thus, the brace according to the invention can be worn 24 hours a day without leading to much pain or distress. The anterior support of the present brace provides suitable anterior forces, for providing a force balance with the forces that are applied to a patient by the posterior thoracolumbar area support during use.

Because of recognition of the body of this more ideal lordotic formation at the thoracolumbar junction, the body is stimulated to an active overcorrection. By this the child diminishes the pressure at the thoracolumbar pads and come free of the sternal support in standing and sitting positions.

In a method for treating a spinal deformity of a patient, such as idiopathic scoliosis, Spondylolisthesis and/or Idiopathic Kyphosis, posterior pressure is being applied to the thoracolumbar area of the patient for treating the spinal deformity.

The innovative idea is, to apply pressure to the thoracolumbar area for treating a spinal deformity. This pressure serves as a spinal support, wherein, preferably, the pressure is mainly directed at supporting the thoracolumbar junction, directly and/or indirectly such as via paraspinal muscles. By applying such pressure, the development of said spinal deformities may surprisingly be halted. Said pressure can also -prevent progression of kyphotic and scoliotic curves. In an aspect, the pressure is mainly directed in such a way in order to restore or protect the thoracolumbar lordosis.

Preferably, the brace is arranged to apply no or relatively little pressure to other spinal areas, particularly the middle lumbar and middle thoracic spine parts, leaving these spinal areas substantially 'free' to move as the patient desires. By leaving these other spinal parts substantially free, the patient may even develop or return the spine into a more natural spinal shape, having a diminishing need to create further abnormal curvatures. This is contrary to the usual methods of brace treatment of spinal deformities, wherein the deformed lumbar and/or thoracic spinal parts are fixated by brace supports for preventing further deformations.

In an aspect of the invention, the brace provides a point of application at a more primary process and not at a secondary process, as the progressive scoliotic and kyphotic curves are thought to be.

Without wishing to be bound to any theory, the innovative idea behind the present invention, to apply pressure mainly to the thoracolumbar area for treating a spinal deformity, is explained in the following paragraphs.

In the proposed underlying theory to the present invention, the cause of the deformities is to be found in a normal adaptation of the spinal column to forces into the body. One of the most important functions of the total spinal column is to protect the central cord for damage not only as a shell against direct forces, but in our opinion also to distracting forces in which a mismatch between length of the cord and the spinal canal plays a keyrole by inducing tension in the system.

Said normal adaptation brings the spine to certain protecting positions in relation to spinal length, spinal tension and position in the spinal canal of the neuromuscular structures. These neuromuscular structures comprise the total spinal cord surrounded by the Dura Mater from the brainstem in relation to the foramen magnum of the skull to the lumbar and sacral roots, and their attachments to muscles in the body axis. The most clearly involved muscles in this matter are believed to be the hamstring muscles of the legs. This is contrary to the above-mentioned general believes concerning causes of Idiopathic Scoliosis and kyphotic deformities, wherein the cause of the deformity is to be sought at the level of he apex of the curve or completely outside the spinal column and its contents.

In the present approach, it is assumed that many spinal deformities are natural adaptations of the body to on a discrepancy in growth or growth potentials between the osseous spinal canal and its neuro(muscular) contents. Therefore, in the present invention, a main goal of treatment is, to facilitate the creation or preservation of a normal thoracolumbar lordosis, by applying posterior pressure to the thoracolumbar area.

The presently proposed theory is further elucidated as follows. At the moment the child starts walking, a lumbar lordosis will start to develop out of the total kyphotic formation of the spine in the foetal and sibling period. If growth or stretch possibilities of the central neuromuscular structures cannot stay at pace with the overall growth of the osseous spinal column at first at the lumbar spine and more important at the thoracolumbar junction, the creation of lordosis will stop or slow down. The latter is caused by the patient him/herself, who automatically will tend to prevent the stretching of the precious neuromuscular structures. This leads to a "flattening" spine in the lower spine area, and to a flattening spine at the thoracolumbar zone. There is also a preference in more kyphotic postures chosen by the children in order to distract the tensile forces in an active way.

Above said "flattening" spine in the lower area, the forces of gravity and the ongoing protection of the body to prevent stretching of the precious neuromuscular structures will give shortening forces to the spine by muscular actions leading to compression on the anterior parts of the thoracic and thoracolumbar spine.

Also, it is believed that at the thoracolumbar zone, the cord and conus medullae change position in the spinal canal from a relative anterior position in the thoracic area to a relative posterior position in the lumbar area. The brace according to an aspect of the invention can also be seen as a protector against unintended forces on the cord and conus medullae in this mobile area.

As a consequence of these forces with their shortening and flattening power, intervertebral discs will gradually show diminished height and herniations of disc material through the endplates, also called Schmorrl's nodi, leading to progressive kyphosis. At the dorsal side of the column, the dorsal structures become absolute or relatively higher than normal and make thoracolumbar kyphosis structural and rigid.

The difference of reaction between the anterior parts of the vertebral column and the posterior parts is lead in the total different anatomical and physiological properties of the structures: mainly bony parts in the posterior part reacting to the more than normal working dorsal muscles to keep the body upright as constant as possible. The bone at the dorsal side will react according to Wolff's Law to these more than normal compressive, and for the total body extending forces with hypertrophy and becoming denser. At the anterior part the vulnerable, to compressive forces totally differently acting structure of the intervertebral disc show diminishing of height, endplate herniations (Schmorl nodi) and incapability of normal outgrow of the endplate apophysis. This gives the vertebra an anterior wedge-shaped form at the end of growth. If there is any asymmetrical construction (very common) of especially the dorsal structures at the thoracolumbar or lumbo-sacral junction, the compressive or shortening forces will lead to a buckling of the spine above or under or at both sides, thus creating scoliotic deformities.

Through this theory, the occurrence of 80% of scoliotic deformity in girls can be explained by their greater laxity in joints and their earlier start of the second growth spurt in adolescence. Besides, applied forces on one of the normal curves in the sagittal plane can produce alterations in different plane than the sagittal plane of the other curvature. A relation between the length of the vertebral canal and the vertebral bodies is described in scoliosis by Porter. By creating scoliotic curves the distance of the canal becomes shorter for the cord.

Besides, it is believed that by applying pressure to the thoracolumbar area as the main brace target zone, and preferably by applying a lordotic force at the thoracolumbar junction, the natural lordosis can be achieved during further child/adolescent growth, so that the thoracolumbar spine can be developed as in a normal spine. Herein, the development of the spine towards a natural spine shape can be achieved by the patient himself, simply during further growth. Therein, the lower spine part will simply follow the lordosis that is applied to the thoracolumbar junction by the brace.

Finally, the presently proposed theory of scoliosis simply being an adaptation during growth in which tension in the whole system of the spine and controlling muscles is abnormal because of a mismatch in growth of the osseous canal and its precious contents may be described, using Mechanics of Materials theory as a starting point, for instance Hookes law. Herein, it is believed, that the so called, commonly known "kwispeleffect" (Dutch) ("tail wagging effect"), as described for instance on pages 156-158 of "Collegedictaat Stijfheid en Sterkte I", prof. dr. ir. A.D. de Pater, fifth edition, 1982, Technische Hogeschool Delft, plays an important role as a model to explain the occurrence of spinal deformation and to describe the effect achieved by the brace according to the invention.

Further embodiments of the invention are described by the dependent claims.

Embodiments of the invention will now be described, by way of example only, with reference to the accompanying schematic drawings in which corresponding reference symbols indicate corresponding parts, and in which:
figure 1 shows a front view of an embodiment of the invention, wherein the brace is in an in an opened position;
figure 2 shows a side view of the embodiment of fig. 1, wherein the brace is in a use position;
figure 3 shows a rear view of the embodiment of fig. 1, wherein the brace is in the use position;
figure 4 schematically shows a perspective front view of a patient wearing the embodiment of fig. 1;
figure 5 shows a side view of the use of the embodiment of fig. 1;
figure 6 shows a rear view of the use of the embodiment of fig. 1;
fig. 7 is a cross-section over line VII-VII of figure 6, depicting the posterior part of the embodiment of fig. 1; and
fig. 8 a cross-section over line VIII-VIII of figure 6.

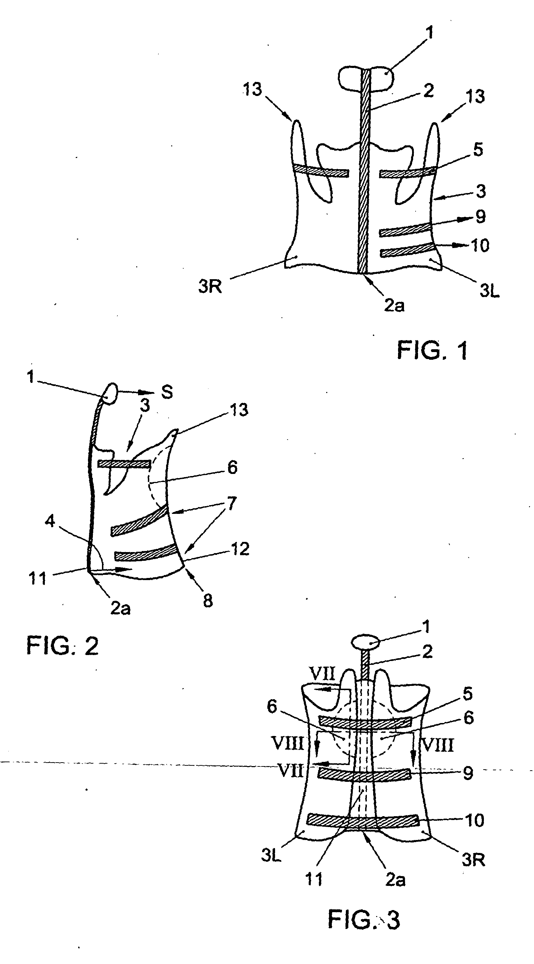

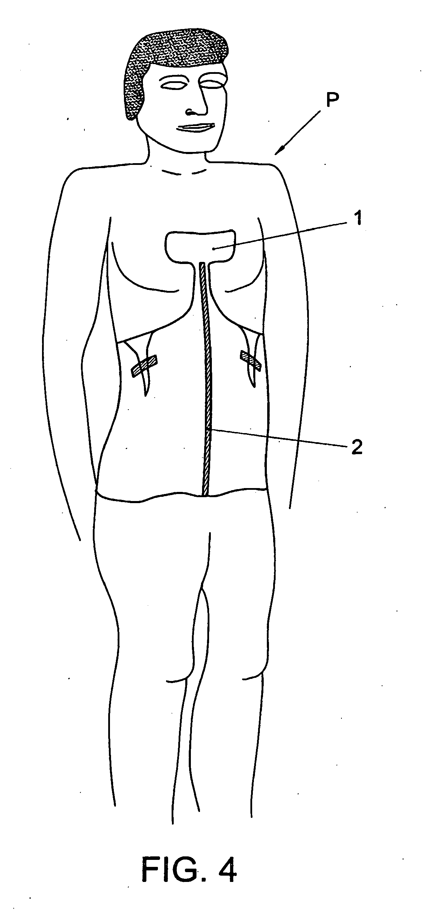

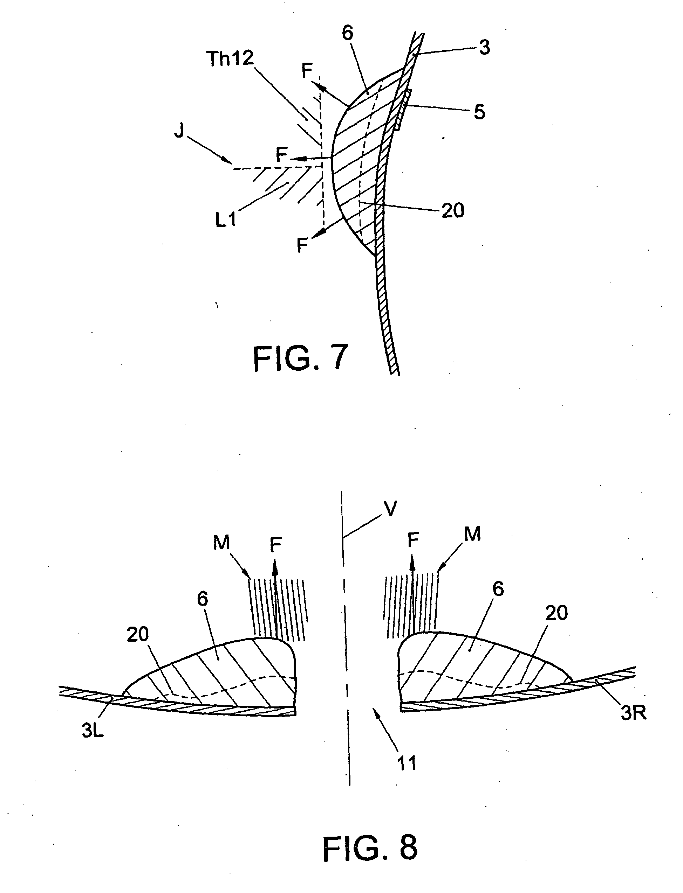

Figures 1-3 show an embodiment of a brace according to the present invention. The brace is arranged for treatment of a spinal deformity of a patient P, for instance for treating Idiopathic Scoliosis. Figures 4-6 shown the brace during use, when it is being worn by the patient P. The present brace embodiment comprises a posterior thoracolumbar area support 6 and an anterior support 1, 2a. The posterior thoracolumbar area support 6 is arranged for applying pressure F to the thoracolumbar area of the patient during use. This thoracolumbar area lies at the commonly known thoracolumbar junction J, which lies between Thoracic Vertebra Th10 and Lumbar Vertebra L2 , measured along the spine. The apex of the applied pads can be located at about Thoracic Vertebra Th12 to Lumbar Vertebra L1 during use. The positions of the last Thoracic Vertabra Th12, the first Lumbar Vertabra L1 and the thoracolumbar junction J with respect to the thoracolumbar area support 6 during use of the present brace, are schematically shown figure 7. The location of said vertebrae is common general knowledge to the skilled person. The posterior, thoracolumbar area 6 support is depicted by dotted lines in figures 1-3. This support, which in the present embodiment actually comprises two separate support pads 3, is also visible in the cross-sections of figures 7 and 8.

Said anterior support 1, 2a of the brace is arranged for applying counter pressures to two separate anterior counter areas when the brace is being worn by the patient P. These two anterior areas are the sternal area and the pelvic bone area. Therefore, the brace of the present embodiment is arranged for providing corrective pressures at three points, i.e. three areas, of the body. The present embodiment of the brace is comfortable during its use, whereas the brace can provide a certain treatment of the deformity, as has been explained above.

In the present embodiment, said anterior support 1, 2a comprises a sternal support 1, which is arranged for applying pressure to said anterior sternal area of the patient P during use. In an embodiment, the sternal support 1 can be made of strong synthetic material (like polypropylene) with a soft liner, for instance having a width of approximately 12 centimetres and a height of approximately 4 cm in the centre and 5 cm at the sides. Naturally, this support may also be provided in various other dimensions. Preferably, the sternal support 1 is arranged to be positioned under the sternoclavicular joints during use. In that case, the sternal support 1 can push the chest backward during use, wherein the sternal support 1 can also create a reminder for the patient P in order to activate him with his dorsal muscles to stay away from the sternal support to prevent painful or sore spots. In that way the patient P is trained actively during use, to create an active extending and lordotic posture especially at the thoracolumbar joint, while standing and sitting at all time they are awake.

The anterior support further comprises a pubic bone support 2a. The pubic bone support is the lower part 2a of a substantially rigid connector 2, which extends ventrally during use (see fig. 4). This rigid connector 2 actually links the pubic bone support 2a and the sternal support 1 rigidly to each other. In an embodiment of the invention, this rigid connector is made of a metal or alloy, for instance Aluminium, Carbon fibre enforced synthetics, Stainless Steel or Memory Metal. In the present embodiment, the rigid connector 2 is a elongated element, for instance a bar or rod. The connector 2 can be fixed to other brace parts 3R, 3L, 1 in various ways, for instance using integral connecting means, rivets and/or the like. The connector 2 can, for example, be covered with polyester woven or other strong but soft material, for example having the same colour as other brace material. Preferably, the brace is arranged to be in good contact with the upper part of the pubic bone and the distal part of the Musculus Rectus Abdominis during use, for instance via the distal end of the elongated connector 2, Preferably, the overall brace is arranged such, that normal sitting, with the hips in a 90° flexion or even some more, is be possible at all times. Preferably, the Ventral Iliac Spines are covered by the brace.

The anterior supports 1, 2a and said thoracolumbar area support 6 are coupled to each other by an intermediate brace part. This intermediate part comprises a left side part 3L and a right side part 3R. The substantially rigid connecting rod 2 extends centrally between these two brace side parts 3L, 3R (see fig. 1). The side parts 3R, 3L can be made, for instance, of strong synthetic material, polypropylene, or the-like, comprising for example a soft liner (for example a foamlike liner).

The overall brace preferably has a definitive and anatomic correct form. The brace may comprise, for instance, prefabricated modules, with a wide range of choice in relation to sizes and measurements at pelvic, waist and chest level of potential users. To the skilled person, for instance orthotist, it will be clear, how such a modular brace can be provided. Preferably, the overall brace is designed to provide an optimal fit for comfortable wearing. Preferably, the brace does not have any sharp rims or the like at any place of the brace in total which is in contact with the skin.

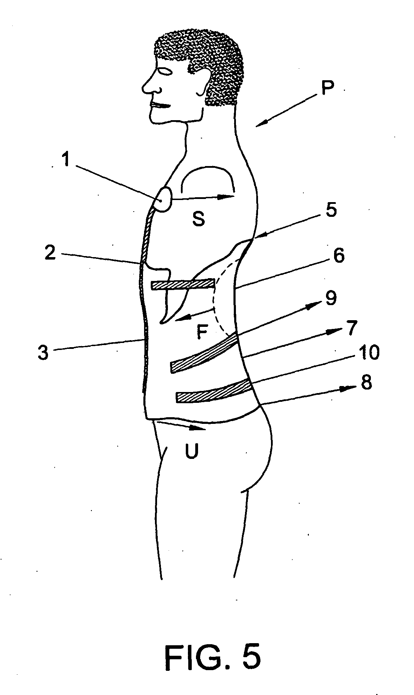

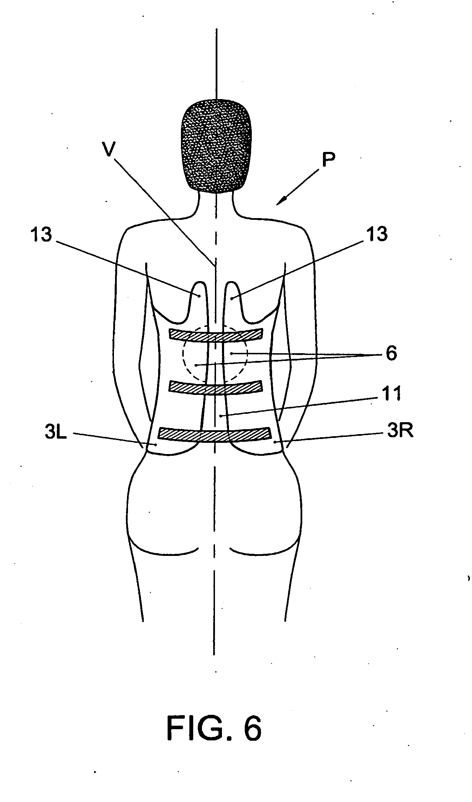

The present brace embodiment is designed for providing a longitudinal, posterior opening 11 during use, for leaving the area of the spinous processes of the spine substantially free (see fig. 6). In the present embodiment, there is already a lordotic form at the front of the brace (convex) and backside thereof (concave), in order to give the belly of the patient P enough space during use, and give the spine the opportunity to follow a more natural lordotic curvature (see fig. 5). Preferably, the primary lordotic curve 7 in the backside of the brace is already in the range of about 40-45° (see figures 3 and 5).

In the present embodiment, the upper rear side parts 13 of the brace (which are parts of said right and left brace parts 3R, 3L) are dimensioned to end about thoracic vertebrae Th9-Th10, at least under the inferior points of the Scapulae (see fig. 6). These upper parts 13 are flared out backwards (see fig. 2) in order to give place for the chest to come backwards by active extension of the spine to get free of the sternal support 1. In the same way, distal ends 8 at the backside of the brace may also be given a flaring out in order to give pelvis and buttocks space to come backwards (see figures 2 and 5).

As is shown in figures 6, 7 and 8, the thoracolumbar area support 6 actually comprises two convex posterior support pads 6, which face the back of the patient during use P. The support pads 6 abut the longitudinal opening 11 of the brace during use. These spaced-apart posterior support pads 6 are arranged for supporting two opposite thoracolumbar paraspinal muscle areas M (see fig. 8). These muscle areas M abut the spine, as is known to a orthotist. Therefore, the thoracolumbar area support pads 6 can support the thoracolumbar junction J of the spine indirectly during use, via said paraspinal muscle areas M, which provides a relatively comfortable spinal support (this contrary to a support, which would put pressure on the spine directly via the spinous processes).

The thoracolumbar support pads 6 can be arranged in various ways. In the present embodiment, the thoracolumbar support pads 6 are attached to the inner side of the brace side parts 3R, 3L, for instance by gluing one or more layers of the same lining material just aside the longitudinal opening 11. The support pads 6 preferably are arranged to offer a relatively smooth, elliptical and symmetric configuration to the back of the patient P, which gives a recognizable and comfortable support. During use, these thoracolumbar support pads 6 give a compressive force F on the Paraspinal muscles M in the thoracolumbar zone. As follows from figures 7 and 8, each of the thoracolumbar pads 6 of the present embodiment has a more acute edge abutting at the longitudinal opening 11, so the most elevated part of the thoracolumbar support is situated at or near the opening 11 (see fig. 8), and preferably substantially in or near the longitudinal centre of each pad (see fig. 7). Also, the supporting sides of the thoracolumbar support pads 6 are more convex than the convex form of the brace parts extending there-below (see fig. 7). Thus, the thoracolumbar support pads 6 are arranged to provide a cantilever zone, such that the brace creates lordosis during use.

Following from the above and the figures, the present embodiment is particularly arranged for applying a lordotic force F to said thoracolumbar area, via said Paraspinal muscles M. This lordotic force is shown by arrows F in figures 7 and 8. The lordotic force F serves as a supporting force for supporting the thoracolumbar area of the spine. Besides, this lordotic force may lead to improvement of natural lordosis in the lumbar spine part, which might be understood by simply following said theory of the "kwispeleffect" which is known by itself from common Mechanics of Materials.

In view of the foregoing and the figures, the present brace embodiment is also arranged to apply said pressure to the thoracolumbar area in a substantially 2-dimensional manner, such that the pressure is mainly directed parallel to the sagittal plane V of the patient P during use (see figures 6-8). Therefore, the brace as such preferably applies only little or no torsion to the spinal column during use. Also, preferably, the main focus of corrective pressure generated by the brace is directed onto the thoracolummbar area, wherein the anterior supports 1, 2a serve for generating the respective counter forces S, U. In that case, the brace applied little or no pressure to other parts of the spinal column, such as the apexes of lumbar and thoracic and cervical spine parts. This leads to a relatively high amount of comfort with respect to the wearing of braces known from the art.

In an aspect of the invention, the substantially rigid connector 2 is adjustable between at least a plurality of positions, for adjusting the amount of pressure applied to said thoracolumbar area. For example, the connector 8 may be bendable backwards, for example by physician or orthotist, or by continuous action by the use of Memory Metal. Therefore, a continuously improving correction can be applied in time, by adjusting the connector 2 to respective treatment positions.

Secondly, adjusting extra layers on the thoracolumbar pads at regular controls at the outpatient clinic of the doctor or the orthotist shop - besides the adjustments of the sternal support 1- offers the possibility to get more correction in the overall period the brace is in use. In figures 7, 8 dotted lines 20 show possible dividing lines between pad layers.

Furthermore, the brace can comprise various means for closing and tightening the brace during use. In the present embodiment, the side parts 3R, 3L of the brace comprise a number of straps 5, 9, 10 for closing and tightening the brace during use. For instance, an upper strap 5 is provided. The upper strap 5 extends behind the thoracolumbar area support 6 during use (see fig. 7). In the present embodiment, the upper strap 5 is attached to the brace with a rivet at the right side. The upper strap 5 is lead to a reversing locking link also attached to the brace by a rivet on the left side and then lead to a same link at the right side. The upper strap 5 provides for an adequate contact of the thoracolumbar support pads 6 to the para-spinal muscles M during use.

The present embodiment further comprises a middle strap 9. This middle strap 9 can, for example, be fixed at the right side at the back of the brace, going to a reversing and locking link at the left side then to a same reversing and locking link at the right side. A long end of the strap 9 with a Velcro attachment at the tip is then lead to the left side again where more at the front it can be adjust to the body of the brace. This middle strap 9 is arranged to close the longitudinal brace opening 11 at the waist, and to provide proper tightening, also for obtaining adequate contact of the thoracolumbar support pads 6 to the para-spinal muscles M, and particularly to create a fulcrum at which the spine comes backward, either passively or actively.

Besides, a lower strap 10 is being provided, which also serves for tightening the brace during use (see figures 5, 6). Both the middle and lower strap 9, 10 are located caudally with respect to the thoracolumbar area support 6.

During use, the brace is being tightened on the patient P as shown in figures 4-6, using the straps 5, 9, 10, such that the sternal support 1 extends in front of the sternal area, the pubic bone area support 2a extends in front of the pubic bone area, and the thoracolumbar support pads 6 extend behind the paraspinal muscle areas M of the thoracolumbar area. Thus, the brace provides treatment of a spinal deformity of the patient P, simply by applying posterior pressure forces F to the thoracolumbar area of the patient P (see fig. 7). Herein, preferably, the posterior pressure is focused near the thoracolumbar spine to give support towards a as-normal-as-possible lordosis in the whole thoracolumbar and lumbar spine particularly, during use, the brace applies a lordotic force to said thoracolumbar area. Besides, a lordosis of more than 30° may applied to the total lumbar area, for instance a lordosis in the range of about 40-45°. Preferably, a smooth cotton or moisture absorbing seamless shirt is worn under the brace by the patient P, for further comfort.

Also, during use, anterior counter pressures are applied. It is advantageous when a first counter pressure S is applied to an upper part of the chest, and/or to a sternal area, wherein a second counter pressure U is applied to a region formed by the pubic bone and the distal parts of the abdominal muscle. Said first and second counter pressure area shown in figures 2, 5 by arrows S respectively U. Thus, said three-point correction is applied to the spine in a relatively comfortable manner.

Herein, a main idea is to prevent deterioration and even correct spinal deformities, by applying correction forces to create and restore a certain amount of lordosis at the thoracolumbar junction of the spine in a natural, symmetrical and therefore comfortable wary. Preferably, lordotic force is used at the thoracolumbar junction as the main target zone in order the achieve the natural lordosis the thoracolumbar spine should have developed in a normal spine. As is shown in figures 4-6, this is simply achieved by the brace, wherein the posterior pressure is being applied by said thoracolumbar area support pads 6. Preferably, no or relatively little pressure is applied to or near other spinal areas, leaving these spinal areas substantially 'free' to move as the patient desires.

Particularly, the present brace applies correcting pressures a substantially 2-dimensional manner, such that the pressure is mainly directed parallel to the sagittal plane V of the patient P. This is contrary to many usual, uncomfortable methods of treating spinal deformities.

Besides, the present brace supports two opposite thoracolumbar paraspinal muscle areas of the patient via said posterior support pads 6. This further enhances comfort, since in this way, substantially no treatment pressure is applied directly to the spinous processes.

Also, preferably, as follows from the above, during use, the brace directs said lordotic enhancing forces F mainly at the thoracolumbar junction J. Particularly, the brace facilitates or creates a natural thoracolumbar lordosis by applying forces F near the thoracolumbar spine in a symmetrical manner with respect to the spine. Also, the use of the brace leads to correction of sagittal alignment of the spine.

As follows from the above and the figures, during use of the present embodiment, said two anterior counter forces S, U are located at the sternal bone, and at the region formed by the pubic bone and the distal parts of the abdominal muscles M respectively. Therefore, also, the creation of a normal thoracolumbar lordosis is facilitated, particularly by applying forces by three point correction in a substantially symmetrical manner. This can be carried out, for example, for treating scoliotic deformities and early or more progressed kyphosis

Prior art braces for treating scoliosis try to give correction in a three dimensional way. The brace according to the present invention utilizes forces, which are preferably directed parallel to the sagittal plane only, for correcting spinal deformities. The brace according to the invention can also prevent the spine to escape into a three-dimensional deformity, in the case of asymmetrical formed dorsal structures at the thoracolumbar and lumbosacral junctions.

Furthermore, preferably, the elongated connecting rod 2 of the brace is adjusted during use, for adjusting the thoracolumbar treatment pressure F of the brace. For instance, at regular moments, the amount of correction can be altered, simply by adjusting the rod 2 on which the sternal support 1 is mounted. Furthermore, preferably, to this aim, as mentioned above, the posterior support pads 6 of the brace can be made thicker at the thoracolumbar junction, for adjusting the treating pressure F.

Besides, by applying said treatment pressure to the thoracolumbar area, the thoracolumbar discs can be protected against compressive forces. In this way, an environment can be created, in which the discs and the endplates with the open apophyses can develop under more physiologic conditions towards a normal form.

Preferably, together with passive correction of the spinal deformity, a program of exercises is started or continued, to create a normal lordosis at the thoracolumbar junction with emphasis on keeping this area movable and strive to a good condition of the musculature (for example the long spinal muscles and shoulder muscles) that is able to support this goal.

In many forms, where in clinical investigation a certain mismatch between the length of the spinal cord and the osseous structures is found, where a clear scoliosis or thoracal hyperkyphosis is not yet present, the brace according to the invention can also be used to protect and realign the thoracolumbar junction. In these cases, untreated, ongoing compensation activities of the spinal musculature are expected to deform the lower lumbar spine into a condition normally called degeneration, especially when encountered in people under the age of 45 years. Particularly, but not exclusively, the brace according to the present invention is directed to treatment of deformities of the spine having a relatively short central cord, such as idiopathic scoliosis, idiopathic kyphosis and other such conditions.

While an specific embodiment of the invention has been described above, it will be appreciated that the invention may be practiced otherwise than as described. The description above is intended to be illustrative, not limiting. Thus, it will be apparent to one skilled in the art that modifications may be made to the invention as described without departing from the scope of the claims set out below.

For instance, the brace can be designed in a range of prefabricated modules to fit various types of bodies. A desired fit and adjustments can be made with respect to the patient, for example, by cutting brace parts and/or by applying extra pads.

Also, to the skilled person it will be clear that the brace can be made of various materials, for example plastics, textiles, metals, memory metal, inflatable pads or devices attached to the inner side of the brace, and the-like, and of various combinations of suitable materials. The brace materials are preferably such that the brace can create a symmetric correction towards a more normal lordosis at the thoracolumbar junction.

Furthermore, for instance in already structural hyperkyphosis with rigidity on correction tests and with an apex above the ninth thoracale vertebra, instead of a sternal support, straps around the shoulders can be used. Such shoulder straps can be, for instance, fixed at the posterior part of the brace. Also in that case, the brace can be arranged on creating a lordotic enhancing force at the thoracolumbar junction, for instance when only a longer lever-arm is adjusted to the brace embodiment for the first period of treatment.

## Claims

1. A brace for treatment of spinal deformities, such as Idiopathic Scoliosis, Idiopathic Kyphosis and/or Spondylolisthesis, the brace at least comprising a posterior support (6) and an anterior support (1, 2a) arranged to apply pressure (F, S, U) to a posterior area and at least one anterior counter-area respectively, **characterised in that** the posterior support is a thoracolumbar area support (6) arranged to apply a lordotic pressure (F) to a thoracolumbar area only, and to apply no or relatively little pressure to other spinal areas than the thoracolumbar area, when the brace is worn by the patient.

2. A brace according to claim 1, wherein the posterior thoracolumbar area support (6) is arranged to apply a lordotic pressure (F) to the thoracolumbar junction between Thoracic Vertebra Th 10 and Lumbar Vertebra L2.

3. A brace according to claim 1 or 2, wherein the posterior thoracolumbar area support (6) has an apex at about Thoracic Vertebra Th 12 and Lumbar Vertebra L1.

4. Brace according to any of the preceding claims, wherein the brace is arranged to apply said pressures (F, S, U) in a substantially 2-dimensional manner, such that the pressures are mainly directed parallel to the sagittal plane (V) of the patient (P) during use.

5. Brace according to any of the preceding claims, wherein the thoracolumbar area support (6) comprises at least one convex posterior support pad.

6. Brace according to claim 5, comprising two spaced apart posterior support pads (6) for supporting two opposite thoracolumbar paraspinal muscle areas (M).

7. Brace according to any of the preceding claims, wherein said anterior support at least comprises a sternal support (1, 2a) for applying pressure (S) to an anterior sternal area.

8. Brace according to claim 7, wherein said anterior support comprises a pubic bone support (2a), for applying pressure to an anterior pubic bone area (U).

9. Brace according to claim 8, wherein said sternal support (1, 2a) and pubic bone part are rigidly linked to each other by a substantially rigid connector (2).

10. Brace according to claim 9, wherein said substantially rigid connector is adjustable between at least a plurality of positions, for adjusting the amount of pressure (F) applied to said thoracolumbar area.

11. Brace according to claim 8 or 9, wherein said substantially rigid connector (2) comprises a metal or alloy bar.

12. Brace according to claims 11, wherein said pubic bone support (2a) is part of the distal end of the bar (2).

13. Brace according to any of the preceding claims, wherein said anterior support (1, 2a) and said thoracolumbar area support (6) are coupled to each other by an intermediate brace part (3R, 3L).

14. Brace according to any of the preceding claims, arranged for providing a longitudinal opening (11), in a use position, for leaving the spine of the patient (P) substantially free.

## Patentansprüche

1. Schiene zur Behandlung von Wirbelsäulenverformungen wie idiophatische Scolisose, idiophatische Kyphose und/oder Spondilolisthesis, wobei die Schiene zumindest eine hintere Stütze (6) und eine vordere Stütze (1, 2a) aufweist, die dafür angeordnet sind, Druck (F, S, U) auf jeweils einen hinteren Bereich und zumindest einen vorderen Gegenbereich auszuüben, **dadurch gekennzeichnet, dass** die hintere Stütze eine Stütze (6) für den Lendenbereich ist, die dafür ungeordnet ist, einen Iordotischen Druck (F) nur auf einen Lendenbereich auszuüben und keinen oder relativ wenig Druck auf andere Wirbelsäulenberelche als die Lendengegend auszuüben, wenn die Schiene von dem Patienten getragen wird.

2. Schiene gemäß Anspruch 1, wobei die hintere Stütze (6) für den Lendenbereich dafür ungeordnet ist, einen Iordotischen Druck auf die thorakolumbale Verbindung zwischen dem Brustwirbel Th 10 und dem Lendenwirbel L2 auszuüben.

3. Schiene gemäß Anspruch 1 oder 2, wobei die hintere Stütze (6) für den Lendenbereich an ungefähr dem Brustwirbel Th 12 und dem Lendenwirbel L1 eine Spitze aufweiset.

4. Schiene gemäß irgendeinem der vorherigen Ansprüche, wobei die Schiene dafür anbeordnet ist, die Drücke (F, S, U) in einer im Wesentlichen zweidimensionalen Art und Weise auszuüben, so dass die Druckkräfte hauptsächlich parallel zu der sagittalen Ebene (V) des Patienten (P) während der Benutzung ausgerichtet sind.

5. Schiene gemäß irgendeinem der vorherigen Ansprüche, wobei die Stütze (6) für den Lendenbereich zumindest ein konvexes hinteres Stützkissen aufweist.

6. Schiene gemäß Anspruch 5 mit zwei voneinander beabstandet angeordneten hinteren Stützkissen (6) zum Stützen von zwei gegenüber liegenden paraspinalen Lendenmuskelbereichen (M).

7. Schiene gemäß irgendeinem der vorherigen Ansprüche, wobei die vordere Stütze zumindest eine Brustbeinstütze (1, 2a) aufweist, um Druck (S) auf einen vorderen Brustbeinbereich auszuüben.

8. Schiene gemäß Anspruch 7, wobei die vordere Stütze eine Schamknochenstütze (2a) aufweist, um Druck auf einen vorderen Schamknochenbereich (U) auszuüben.

9. Schiene gemäß Anspruch 8, wobei die Brustbeinstütze (1, 2a) und das Schamknochenteil fest miteinander durch ein Im Wesentlichen steifes Verbindungstell (2) verbunden sind.

10. Schiene gemäß Anspruch 9, wobei das Im Wesentlichen steife Verbindungsteil zwischen zumindest einer Vielzahl von Positionen einstellbar ausgebildet ist, um die auf den Lendenbereich ausgeübte Druckstärke (F) einzustellen.

11. Schiene gemäß Anspruch 8 oder 9, wobei das im Wesentlichen steife Verbindungsteil (2) eine Stange aus Metall oder einer Legierung aufweist.

12. Schiene gemäß Anspruch 11, wobei die Schamknochenstütze (2a) Teil des entfernt liegenden Endes der Stange (2) ist.

13. Schiene gemäß irgendeinem der vorherigen Ansprüche, wobei die vordere Stütze (1, 2a) und die Stütze (6) für den Lendenbereich durch ein dazwischen liegendes Schienenteil (3R, 3L) aneinander gekoppelt sind.

14. Schiene gemäß Irgendeinem der vorherigen Ansprüche, die dafür angeordnet ist, in einer Benutzungsposition eine längliche Öffnung (11) bereitzustellen, um die Wirbelsäule des Patienten (P) im Wesentlichen frei zu lassen.

## Revendications

1. Appareil orthopédique pour le traitement de déformations spinales, comme la scoliose idiopathique, la cyphose idiopathique et/ou le spondylolisthésis, l'appareil orthopédique comprenant au moins un support postérieur (6) et un support antérieur (1, 2a) agencés pour appliquer une pression (F, S, U) respectivement sur une région postérieure et au moins une région antérieure correspondante, **caractérisé en ce que** le support postérieur est un support de région thoraco-lombaire (6) adapté pour appliquer une pression lordotique (F) uniquement sur une région thoraco-lombaire, et pour appliquer peu ou pas de pression aux régions rachidiennes autres que la région thoraco-lombaire, quand l'appareil orthopédique est porté par le patient.

2. Appareil orthopédique selon la revendication 1, dans lequel le support de région thoraco-lombaire postérieur (6) est adapté pour appliquer une pression lordotique (F) à la jonction thoraco-lombaire entre la vertèbre thoracique Th 10 et la vertèbre lombaire L2.

3. Appareil orthopédique selon la revendication 1 ou 2, dans lequel le support de région thoraco-lombaire postérieur (6) a un sommet situé près de la vertèbre thoracique Th 12 et de la vertèbre lombaire L1.

4. Appareil orthopédique selon l'une quelconque des revendications précédentes, dans lequel l'appareil orthopédique est adapté pour appliquer lesdites pressions (F, S, U) d'une manière substantiellement bidimensionnelle, de sorte que les pressions sont principalement orientées parallèlement au plan sagittal (V) du patient (P) en utilisation.

5. Appareil orthopédique selon l'une quelconque des revendications précédentes, dans lequel le support de région thoraco-lombaire (6) comprend au moins un coussinet de support postérieur convexe.

6. Appareil orthopédique selon la revendication 5, comprenant deux coussinets de support postérieur espacés (6) pour supporter deux régions musculaires paraspinales thoraco-lombaires opposées (M).

7. Appareil orthopédique selon l'une quelconque des revendications précédentes, dans lequel ledit support antérieur comprend au moins un support sternal (1, 2a) pour appliquer une pression (S) sur une région sternale antérieure.

8. Appareil orthopédique selon la revendication 7, dans lequel ledit support antérieur comprend un support d'os pubien (2a), pour appliquer une pression sur une région d'os pubien antérieure (U).

9. Appareil orthopédique selon la revendication 8, dans lequel ledit support sternal (1, 2a) et la partie d'os pubien sont liés de façon rigide l'un à l'autre par un connecteur substantiellement rigide (2).

10. Appareil orthopédique selon la revendication 9, dans lequel ledit connecteur substantiellement rigide est réglable entre au moins une pluralité de positions, pour régler le niveau de pression (F) appliqué à ladite région thoraco-lombaire.

11. Appareil orthopédique selon la revendication 8 ou 9, dans lequel ledit connecteur substantiellement rigide (2) comprend une barre en métal ou en alliage.

12. Appareil orthopédique selon la revendication 11, dans lequel ledit support d'os pubien (2a) fait partie de l'extrémité distale de la barre (2).

13. Appareil orthopédique selon l'une quelconque des revendications précédentes, dans lequel ledit support antérieur (1, 2a) et ledit support de région thoraco-lombaire (6) sont accouplés l'un à l'autre au moyen d'une partie d'appareil intermédiaire (3R, 3L).

14. Appareil orthopédique selon l'une quelconque des revendications précédentes, prévu pour fournir une ouverture longitudinale (11), dans une position d'utilisation, pour laisser la colonne vertébrale du patient (P) substantiellement libre.
